Europäisches Patentamt

European Patent Office

Office européen des brevets

(11) Numéro de publication: **0 166 635**
**A1**

## (12) DEMANDE DE BREVET EUROPEEN

(21) Numéro de dépôt: 85400994.1

(22) Date de dépôt: 21.05.85

(51) Int. Cl.⁴: **H 04 N 5/32**

(30) Priorité: 25.05.84 FR 8408253

(43) Date de publication de la demande: 02.01.86
Bulletin 86/1

(84) Etats contractants désignés: DE IT NL

(71) Demandeur: THOMSON-CGR, 13, square Max-Hymans,
F-75015 Paris (FR)

(72) Inventeur: Yedid, Henri, THOMSON-CSF SCPI 173, bld
Haussmann, F-75379 Paris Cedex 08 (FR)
Inventeur: Audoin, Maurice, THOMSON-CSF
SCPI 173, bld Haussmann, F-75379 Paris Cedex 08 (FR)

(74) Mandataire: Grynwald, Albert et al, THOMSON-CSF
SCPI 173, Bld Haussmann, F-75379 Paris Cedex 08 (FR)

(54) **Procédé de traitement d'images radiologiques.**

(57) Procédé de traitement d'images en vue de reconstituer
une image composite de plus grande dimension que les images de base.

L'invention consiste à prendre (15) et numériser (17) un
certain nombre d'images de base pour plusieurs positions
relatives entre la table porte-patient (11) et une unité radiologique (12) à mémoriser ces images dans une mémoire ($M_0$)
et à reconstituer une image composite, par exemple en
transférant des parties choisies des images de base dans
une mémoire de regroupement ($M_1$).

Application à la radiologie du système sanguin.

# PROCEDE DE TRAITEMENT D'IMAGES RADIOLOGIQUES

L'invention concerne un procédé de traitement d'images radiologiques ayant principalement pour but de reconstituer une image composite dont l'une des dimensions au moins est notablement plus grande que le champ de visualisation du récepteur utilisé, ce dernier étant par exemple un amplificateur de luminance connu en soi. L'invention trouve un domaine d'application privilégié en angiographie notamment pour visualiser au moins une partie importante du faisceau et de l'arborescence circulatoire d'un patient.

En angiographie, il est de la plus grande utilité de pouvoir visualiser sur un seul document une partie importante du système artériel et/ou veineux, par exemple notamment la totalité du système artériel et veineux inférieur. On a par le passé pratiqué ces examens à partir d'une installation comportant une source radiogène placée très haut au-dessus du patient pour irradier la totalité de la région à visualiser, ainsi qu'un mécanisme à déplacement de film adapté pour prendre une série de clichés adjacents correspondant à des portions différentes de cette région. Ce système présente le double inconvénient d'un coût d'exploitation élevé car la quantité de film photographique consommée est importante et aussi d'une irradiation très importante du patient. De plus, la lecture du document n'est pas toujours facile en raison des variations de qualité des images successives. Plus récemment, avec l'apparition des amplificateur de luminance qui tendent à remplacer les films, on a proposé de prendre plusieurs clichés pour différentes positions relatives entre l'ensemble source-récepteur et la table porte-patient et de reproduire ces clichés les uns à côté des autres sur un document photographique unique de dimensions nettement plus réduites que la série de clichés décrite ci-dessus, pour une résolution équivalente de l'image. En outre, la dose de rayonnement X appliquée au patient est notablement réduite. Cependant, ce type de présentation implique un effort de reconstruction mentale de l'image, pour le médecin, ce qui est ressenti comme une contrainte.

L'invention concerne un perfectionnement du second type de procédé d'élaboration de document radiologique décrit ci-dessus.

Plus précisément, l'invention concerne donc un procédé de traitement d'images radiologiques prises pour différentes positions relatives entre un ensemble source-récepteur et un support d'un corps à radiographier, le long d'un trajet prédéterminé, du type consistant à mémoriser chaque image sous forme d'informations numériques représentatives de pixels desdites images, caractérisé en ce qu'il consiste à prendre des images en chevauchement, à repérer et mémoriser lesdites positions relatives correspondantes, à lire des parties des informations numériques de certaines images, éventuellement après traitement, lesdites parties étant fonction desdites positions relatives, et à convertir les informations numériques desdites parties en signaux vidéo pour visualiser une image composite constituée de la réunion d'au moin certaines images radiologiques précitées.

Pour la visualisation de l'image composite reconstituée, à partir du moniteur de télévision, il suffit de faire défiler lentement cette image composite sur le téléviseur.

Les éléments de base de l'invention définie ci-dessus permettent en outre la mise en oeuvre d'une nouvelle méthode d'élaboration d'image composite utilisant les habituelles techniques de soustraction logarithmique d'images où les prises de "masques" et d'images à traiter sont effectuées en alternance au fur et à mesure de la progression du produit de contraste.

Plus précisément, l'invention concerne donc aussi un procédé de traitement d'images d'un corps à l'intérieur duquel un produit de contraste s'écoule approximativement dans la direction du trajet parallèlement au trajet précité, procédé selon la définition qui précède et caractérisé en ce qu'il consiste à effectuer un unique balayage entre ledit ensemble source-récepteur et le support dudit corps le long dudit trajet prédéterminé, à prendre et mémoriser deux images pour chaque position relative précitée, l'une en l'absence de produit de contraste dans le champ de ladite image et l'autre en présence de produit de contraste dans une partie dudit champ, à soustraire, de façon connue en soi, les deux images prises à chaque position relative et à lire les parties précitées des informations numériques des images résultant de ces soustractions.

Bien entendu, ce processus peut subir de nombreuses variantes, il est notamment possible de reconstituer d'abord les deux images composites, l'une regroupant les "masques" ou images prises sans produit de constraste et l'autre regroupant les images avec produit de contraste, avant d'effectuer la soustraction des deux images composites et de visualiser le résultat.

L'invention sera mieux comprise et d'autres avantages de celle-ci apparaîtront mieux à la lumière de la description qui va suivre d'un mode de réalisation préféré des moyens de mise en oeuvre de l'invention, faite en référence aux dessins annexés dans lesquels :

- la figure 1 est un schéma-bloc général d'une installation de radiologie mettant en oeuvre le procédé de l'invention ;

- la figure 2 illustre le traitement entre deux images radiologiques voisines ; et

- la figure 3 illustre un traitement complet d'images, conforme à l'invention.

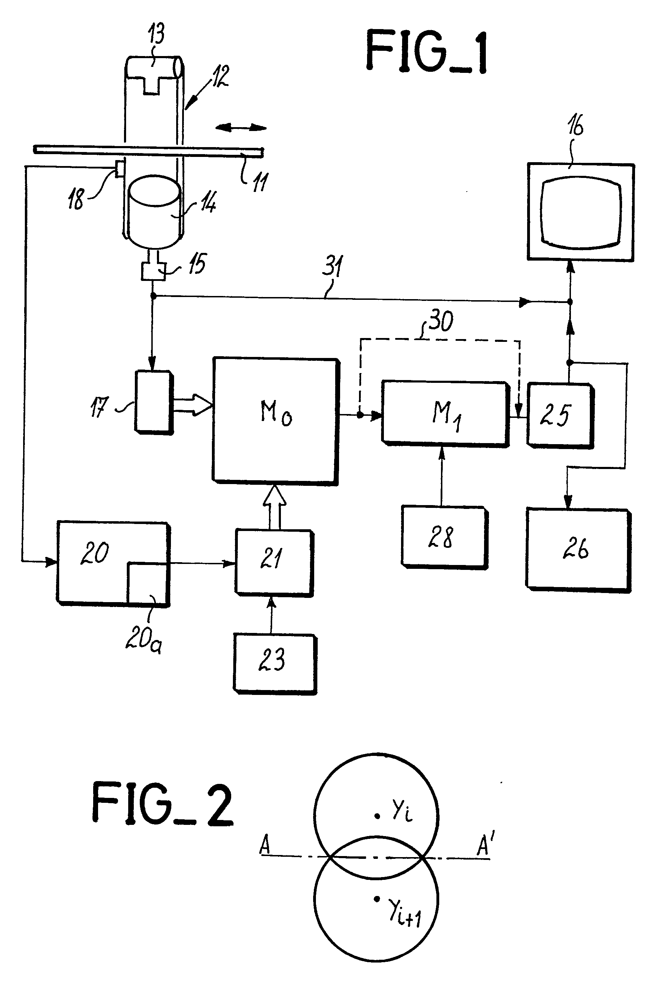

L'installation de la figure 1 comporte une table porte-patient 11 et une unité radiologique 12 comprenant une source de rayons X 13 placée en regard d'un amplificateur de luminance 14 constituant le récepteur d'image radiologique précité. L'unité radiologique 12 et/ou la table 11 sont mobiles en translation le long d'un trajet rectiligne prédéterminé parallèle à la table. L'amplificateur de luminance 14 est lu par une caméra de télévision 15 dont la sortie vidéo est reliée à un moniteur de télévision 16 (liaison directe 31) et à un convertisseur Analogique-Numérique 17 dont la sortie numérique est couplée à une mémoire d'images $M_0$. Des moyens de mesure 18 du déplacement relatif entre la table 11 et l'unité radiologique 12 sont reliés à une unité de calcul 20 qui pilote un générateur d'adresses 21 commandant la lecture de parties choisies de la mémoire $M_0$. Le générateur d'adresses 21 est aussi couplé à un clavier de sélection 23 par lequel l'opérateur peut sélectionner un certain nombre d'images préenregistrées dans la mémoire $M_0$ et commander leur lecture, ligne par ligne et pixel par pixel, dans un ordre prédéterminé. La mémoire $M_0$ est couplée à une mémoire de regroupement $M_1$ dans laquelle certaines images ou parties d'images sont susceptibles d'être transférées sous la commande du générateur d'adresses 21 et du clavier 23. La sortie de la mémoire $M_1$ est reliée à un convertisseur Numérique-Analogique 25 dont la sortie pilote le moniteur 16 et un moyen de reprographie 26 constitué ici de préférence par un dispositif de restitution d'images vidéo à impression laser, connu en soi. On pourra par exemple utiliser un dispositif de ce genre proposé par la firme SORO.

Tous les moyens qui viennent d'être décrits sont individuellement connus à l'exception de l'unité de calcul 20 qui sera décrite plus en détail ci-dessous. Cette dernière reçoit, par les moyens de mesure 18, des informations représentatives du positionnement relatif entre la table 11 et l'unité 12, aux moments des prises d'images. Ces informations peuvent être mises en mémoire dans une unité de mémorisation $20_a$ et l'unité de calcul 20 est agencée et programmée pour piloter le générateur d'adresses 21 de façon à lire sélectivement la mémoire $M_0$ pour ne relever que les informations représentatives des pixels situés dans des régions spécifiques des différentes images en fonction des informations stockées dans la mémoire $20_a$.

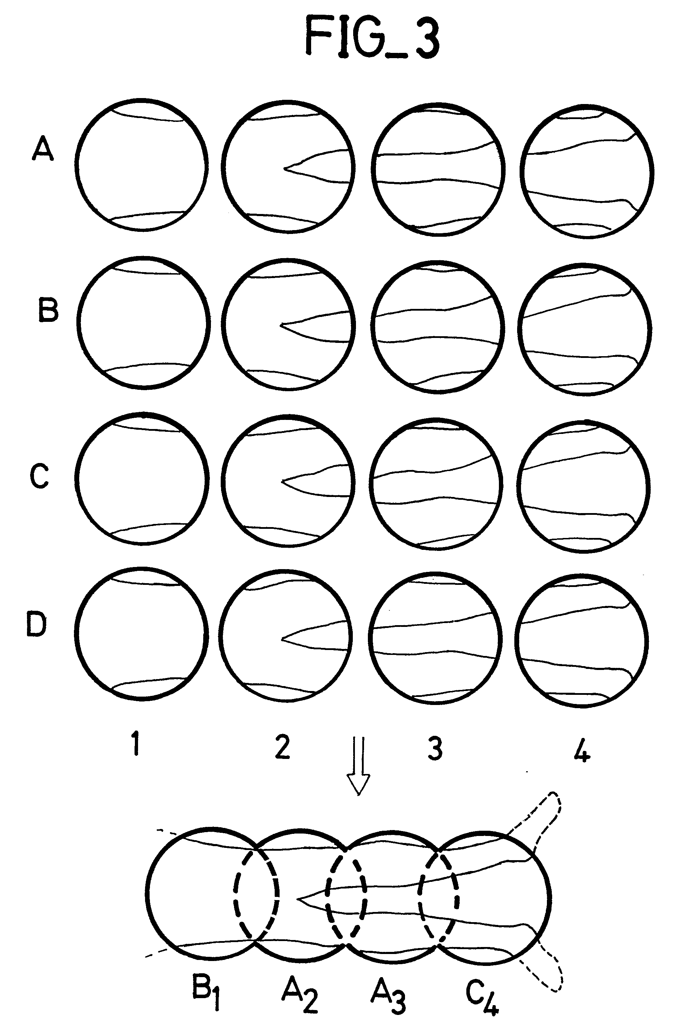

La figure 2 illustre cette lecture sélective. L'amplificateur de luminance délivre traditionnellement une image circulaire caractérisée par la position de son centre Yi et par son diamètre constant. Pour toutes les images relevées et mémorisées, les positions de ces centres Yi par rapport à une origine commune sont mémorisées (mémoire $20_a$) et le diamètre de chaque image étant connu et préprogrammé, il est facile, à l'aide de ces seules informations, de connaître les valeurs de chevauchement entre images voisines, pour déterminer quels sont les pixels à lire dans chaque partie de la mémoire $M_0$ où sont inscrites les informations d'une image donnée, pour les transmettre vers la mémoire $M_1$. Par exemple, si les images sont lues ligne par ligne (ce qui correspond au mode d'affichage par balayage, classique, du moniteur de télévision 16) et si les lignes de deux images voisines passant par les centres de ces images correspondent respectivement aux positions Yi et Yi+1 le long du trajet précité, la ligne AA' joignant les deux points d'intersection des limites des images le long du même trajet correspondra à la position $\frac{Yi+ Yi+1}{2}$ et sera la ligne de séparation à adopter entre les deux images. Il suffira donc de prélever les informations numériques des lignes de l'image vidéo située d'un côté de cette ligne AA' dans une image (figure 2) et les informations numériques des lignes situées de l'autre côté de cette même ligne AA' dans l'autre image adjacente et de transférer ces informations sélectionnées dans la mémoire $M_1$ pour reconstituer ainsi l'image composite recherchée à partir de plusieurs images élémentaires adjacentes.

Il résulte de ce qui précède que la conception du circuit de calcul 20 est à la portée de l'homme du métier puisqu'il se résume à des moyens de calcul simples recevant les valeurs stockées dans la mémoire 20a et des moyens de commande du générateur d'adresses 21. Structurellement, la mémoire 20a peut être une partie de la mémoire $M_0$ tandis que le circuit de calcul 20 peut être câblé ou réalisé par un sous-programme. La mémoire $M_1$ est lue cycliquement en étant pilotée par un générateur d'adresses 28 (mémoire de rafraîchissement) pour permettre un affichage permanent sur le moniteur de télévision 16, après conversion des informations numériques (convertisseur Numérique-Analogique 25) en signal vidéo. Comme la capacité de la mémoire $M_1$ est celle de plusieurs images de la dimension du moniteur de télévision, le générateur d'adresses 28 est programmé pour provoquer un décalage de une ou plusieurs lignes à chaque lecture de façon que la totalité de l'image composite défile lentement sur l'écran du moniteur de télévision 16. La mémoire $M_1$ peut aussi être supprimée si le générateur d'adresses 21 est programmé pour lire successivement les parties utiles de l'image affichée à chaque instant directement dans la mémoire $M_0$. Il est aussi parfaitement possible, sans sortir du cadre de l'invention, de choisir d'autres limites géométriques entre les images en chevauchement. On peut par exemple prendre en compte tous les pixels de la première image puis les pixels de la seconde image qui se trouvent à l'extérieur du périmètre de la première image, et ainsi de suite...... le circuit de calcul est alors modifié pour déterminer et traduire en informations numériques l'équation de l'arc de cercle marquant la limite entre les deux images et pour sélectionner les pixels par un classement des adresses de ces derniers par rapport à cette frontière courbe. Bien entendu, le procédé est transposable de façon évidente pour la reconstruction d'images composites à partir d'images carrées ou rectangulaires.

La figure 3 illustre une mise en oeuvre avantageuse du procédé de l'invention, faisant intervenir plusieurs séquences de prise d'image et de mémorisation correspondant à des balayages successifs entre la table 11 et l'unité radiologique 12. On suppose par exemple qu'on a ainsi réalisé quatre groupes A,B,C,D comportant chacun quatre images en chevauchement, chaque groupe reconstituant ainsi toute la partie inférieure du corps d'un patient pour un examen complet de l'arborescence circulatoire. Ces différentes images sont prises approximativement aux mêmes positions (ces positions étant au nombre de 4 dans l'exemple représenté) le long du trajet de déplacement et elles sont mémorisées dans la mémoire $M_0$.

L'opérateur a ensuite la faculté de les visualiser une par une grâce à une liaison directe 30 commutable entre la sortie de la mémoire $M_0$ et le convertisseur Numérique-Analogique 25. Il peut ainsi sélectionner dans chaque groupe les images les plus exploitables et transférer les informations correspondantes dans la mémoire $M_1$ tout en effectuant les "découpages" résultant du procédé défini ci-dessus. Ainsi, selon l'exemple représenté, l'opérateur a successivement sélectionné les images $B_1, A_2, A_3$ et $C_4$. Bien entendu, en l'absence de mémoire $M_1$ les informations numériques sélectionnées peuvent être lues cycliquement comme mentionné précédemment, directement dans la mémoire $M_0$. Indépendamment de la visualisation de l'image composite contenue dans la mémoire $M_0$ en défilement lent sur l'écran du moniteur de télévision 16, les moyens de reprographie 26 peuvent être mis en oeuvre à tout moment pour délivrer instantanément un tirage sur papier de la même image.

L'invention couvre aussi une application supplémentaire mettant en oeuvre les techniques bien connues de traitement d'images radiologiques par soustraction de deux images, l'une des images ou "masque" étant prise sans produit de contraste (c'est-à-dire avant l'arrivée de ce produit de contraste dans le champ du récepteur 14) et l'autre image étant prise après l'arrivée du produit de contraste dans ce champ. Les moyens de traitement par soustraction peuvent être intégrés à l'installation de la figure 1 sans difficulté. On commence par injecter une dose de produit de contraste dans le patient et on commande ensuite un unique balayage entre l'unité radiologique 12 et la table 11 en prenant chaque fois deux images pour chaque position choisie. Avant d'enregistrer toute image, le champ d'observation correspondant à cette image est observé en permanence en régime de radioscopie (liaison directe 31 entre la sortie de la caméra 15 et le moniteur de télévision 16). Après injection du produit, on prend et mémorise la première image, de préférence peu de temps avant que ledit produit de contraste pénètre dans le champ de l'image observée, puis on prend et mémorise une seconde image dans la même position lorsque le produit de contraste s'est répandu dans une certaine partie du champ de l'image. Une soustraction de ces deux images prises à la même position permet d'améliorer la visualisation de l'arborescence circulatoire dans la partie correspondant à la présence du produit de contraste. Cette partie est mémorisée dans la mémoire $M_1$. On recherche ensuite une nouvelle position relative entre l'unité radiologique et la table pour que l'image visualisée en radioscopie soit sensiblement exempte de produit de contraste et on recommence les mêmes opérations pour transférer une autre partie de l'image composite traitée dans la mémoire $M_1$, et ainsi de suite... Bien endendu, certaines opérations peuvent être inversées. Par exemple, on peut parfaitement reconstituer une image composite complète de "masque" (à partir de toutes les images prises sans produit de contraste qui ont été mémorisées pendant la séquence) et une image composite complète avec produit de contraste, avant de soustraire ces deux images composites l'une de l'autre.

## REVENDICATIONS

1. Procédé de traitement d'images radiologiques prises pour différentes positions relatives entre un ensemble source-récepteur (12) et un support (11) d'un corps à radiographier, le long d'un trajet prédéterminé, du type consistant à mémoriser $(M_0)$ chaque image sous forme d'informations numériques représentatives de pixels desdites images, caractérisé en ce qu'il consiste à prendre des images en chevauchement, à repérer et mémoriser lesdites positions relatives correspondantes, à lire des parties des informations numériques de certaines images, éventuellement après traitement, lesdites parties étant fonction desdites positions relatives et à convertir les informations numériques (25) desdites parties en signaux vidéo pour visualiser (16,26) une image composite constituée de la réunion d'au moins certaines images radiologiques précitées.

2. Procédé de traitement d'images radiologiques selon la revendication 1, caractérisé en ce qu'il consiste à effectuer plusieurs balayages entre ledit ensemble source-récepteur (12) et un support (11) dudit corps le long dudit trajet prédéterminé à prendre et mémoriser au cours de balayages successifs des séries d'images (A,B,C,D) respectives et à sélectionner une suite d'images en recouvrement dans lesdites séries d'images avant de lire les parties précitées des informations numériques de chaque image de ladite suite.

3. Procédé de traitement d'images selon la revendication 1 ou 2, caractérisé en ce qu'il consiste à appliquer les signaux vidéo de ladite image composite à des moyens de reprographie (26).

4. Procédé de traitement d'images radiologiques d'un corps à l'intérieur duquel un produit de contraste s'écoule approximativement dans la direction dudit trajet, selon l'une des revendications 1 à 3, caractérisé en ce qu'il consiste à effectuer un unique balayage entre ledit ensemble source-récepteur (12) et le support (11) dudit corps le long dudit trajet à prendre et mémoriser ($M_0$) deux images pour chaque position relative précitée, l'une en l'absence de produit de contraste dans le champ de ladite image et l'autre en présence de produit de contraste dans une partie au moins dudit champ, à soustraire, de façon connue en soi, les deux images prises à chaque position relative et à lire les parties précitées des informations numériques des images résultant de ces soustractions.

5. Procédé de traitement d'images radiologiques d'un corps à l'intérieur duquel un produit de contraste s'écoule approximativement dans la direction dudit trajet, selon l'une des revendications 1 à 3, caractérisé en ce qu'il consiste à effectuer un unique balayage entre ledit ensemble source-récepteur (12) et le support (11) dudit corps le long dudit trajet à prendre et mémoriser ($M_0$) deux images pour chaque position relative précitée, l'une en l'absence de produit de contraste dans le champ de ladite image et l'autre en présence de produit de contraste dans une partie au moins dudit champ, à lire les parties précitées des informations numériques représentatives de ces deux types d'images, respectivement, pour reconstituer deux images composites précitées et à soustraire, de façon connue en soi les deux images composites l'une de l'autre.

# FIG_1

# FIG_2

FIG_3

Office européen
des brevets

**RAPPORT DE RECHERCHE EUROPEENNE**

## DOCUMENTS CONSIDERES COMME PERTINENTS

| Catégorie | Citation du document avec indication, en cas de besoin, des parties pertinentes | Revendication concernee | CLASSEMENT DE LA DEMANDE (Int. Cl.4) |
|---|---|---|---|
| A | US-A-4 281 249  (LAPIDUS)<br>* Colonne  1, ligne 46 - colonne 2, ligne 56 * | 1,3 | H 04 N    5/32 |
| | --- | | |
| A | EP-A-0 047 177  (ELSCINT LTD.) | | |
| | --- | | |
| A | US-A-4 012 636  (ENGDAHL) | | |
| | --- | | |
| A | FR-A-2 052 548  (SIEMENS A.G.) | | |
| | ----- | | |

DOMAINES TECHNIQUES
RECHERCHES (Int. Cl.4)

H 04 N
A 61 B
H 05 G
G 01 T
G 01 C

Le présent rapport de recherche a été établi pour toutes les revendications

| Lieu de la recherche<br>LA HAYE | Date d'achèvement de la recherche<br>12-09-1985 | Examinateur<br>CRISTOL Y. |
|---|---|---|